(19) Europäisches Patentamt
European Patent Office
Office européen des brevets

(11) **EP 0 928 287 B1**

(12) **FASCICULE DE BREVET EUROPEEN**

(45) Date de publication et mention de la délivrance du brevet:
**01.10.2003 Bulletin 2003/40**

(21) Numéro de dépôt: **98941464.4**

(22) Date de dépôt: **27.07.1998**

(51) Int Cl.[7]: **C07C 381/10**, C07C 311/48, C07F 7/18, C08K 5/36, G02F 1/15, H01M 6/16

(86) Numéro de dépôt international:
**PCT/FR98/01663**

(87) Numéro de publication internationale:
**WO 99/005100 (04.02.1999 Gazette 1999/05)**

(54) **COMPOSES IONIQUES AYANT UNE CHARGE ANIONIQUE DELOCALISEE, LEUR UTILISATION COMME COMPOSANTS DE CONDUCTEURS IONIQUES OU DE CATALYSEUR**

IONISCHE VERBINDUNGEN MIT DELOKALISIERTER ANIONISCHER LADUNG, IHRER VERWENDUNG ALS KOMPONENTEN VON IONENLEITERN ODER VON KATALYSATOREN

IONIC COMPOUNDS WITH DELOCALIZED ANIONIC CHARGE, THEIR USE AS COMPONENTS OF IONIC CONDUCTORS OR CATALYST

(84) Etats contractants désignés:
**DE FR GB IT**

(30) Priorité: **25.07.1997 CA 2211465**

(43) Date de publication de la demande:
**14.07.1999 Bulletin 1999/28**

(73) Titulaires:
- **ACEP INC.**
  **Montreal, Quebec H2Z 1A4 (CA)**
- **UNIVERSITE DE MONTREAL**
  **Montréal, Québec H3C 3J7 (CA)**
- **CENTRE NATIONAL DE LA RECHERCHE SCIENTIFIQUE (CNRS)**
  **75016 Paris (FR)**
- **Institute of Organic Chemistry**
  **Kiev-94, 253660 (UA)**

(72) Inventeurs:
- **ARMAND, Michel**
  **Montréal, Québec H3T 1N2 (CA)**
- **MICHOT, Christophe**
  **F-38000 Grenoble (FR)**
- **YAGUPOLSKII, Yurii**
  **Kiev, 252005 (UA)**
- **YAGUPOLSKII, Lev**
  **Kiev, 252042 (UA)**
- **BEZDUDNY, Andrej**
  **Kiev, 252057 (UA)**
- **KONDRATENKO, Natalya**
  **Kiev, 253154 (UA)**

(74) Mandataire: **Sueur, Yvette et al**
**Cabinet SUEUR & L'HELGOUALCH,**
**109, boulevard Haussmann**
**75008 Paris (FR)**

(56) Documents cités:
- **E.S. LEVCHENKO, ET AL.: "Arenesulphinamidines" JOURNAL OF ORGANIC CHEMISTRY OF THE USSR. (ZHURNAL ORGANICHESKOI KHIMII), vol. 4, no. 1, janvier 1968, pages 144-148, XP002086722 New York, US**
- **D. VAN LEUSEN, ET AL.: "Synthesis of arylsulphonamidoyl fluorides and their use in the preparation of (arylsulphonimidoyl)methyl isocyanides. Partial resolution of optically acitve S-phenyl-N-tosylsulphonimidoyl fluoride" RECUEIL DES TRAVAUX CHIMIQUES DES PAYS-BAS, vol. 103, no. 2, février 1984, pages 41-45, XP002086723 Amsterdam, NL**
- **CHEMICAL ABSTRACTS, vol. 54, no. 20, 25 octobre 1960 Columbus, Ohio, US; abstract no. 20948f, A.V. KIRSANOV, ET AL.: "Derivatives of nitrobenzenesulphonomido-N-phosphoric acids" XP002086725 & ZHURNAL OBSHCHEI KHIMII, vol. 29, 1959, pages 4048-4891,**
- **P. BURK, ET AL.: "Superacidity of neutral Brönsted acids in gas phase" JOURNAL OF COMPUTATIONAL CHEMISTRY, vol. 17, no. 1, 1996, pages 30-41, XP002086724 New York, US**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 928 287 B1

Remarques:

Le dossier contient des informations techniques présentées postérieurement au dépôt de la demande et ne figurant pas dans le présent fascicule.

## Description

La présente invention a pour objet des composés ioniques comprenant une charge anionique fortement délocalisée, un procédé pour leur préparation, ainsi que leurs applications.

Il est bien connu que les sels des acides forts de type $HClO_4$, $HBF_4$, $HPF_6$, $HR_FSO_3$ ($R_F$ radical perfluoré) présentent des propriétés dans le domaine de l'électrochimie et de la catalyse, mais que ces propriétés sont limitées. On connaît par ailleurs les "superacides" obtenus par addition d'un acide de Lewis tel que $SbF_5$ aux composés précités. Ces composés ne sont cependant pas stables autrement que sous forme protonée et dans des milieux non solvatants tels que les hydrocarbures aliphatiques. Les sels sont instables dans les solvants polaires usuels.

Depuis peu, les dérivés des perfluorosulfonimides $H[R_FSO_2NSO_2R_F]$ ($R_F$ = perfluoroalkyle) ont été étudiés. Il présentent des propriétés de stabilité intéressantes sous forme protonée ou sous forme de sels et sont utilisés comme solutés dans l'électrochimie et comme catalyseurs. Toutefois, il n'est pas possible de conférer à ces sels toutes les propriétés requises pour toutes les applications, en particulier en termes d'acidité, de dissociation ou de solubilité, car l'utilisation de composés contenant des chaînes perfluorées de plusieurs carbones n'augmente que peu l'acidité ou la dissociation des sels, par rapport au composé le plus simple $R_F = CF_3$, et induit une rigidité de la molécule au détriment des propriétés de conductivité. Les longues chaînes fluorées sont à la fois hydrophobes et oléophobes et ne permettent pas d'augmenter la solubilité dans les mileux organiques d'une façon notable. Le remplacement de groupements $R_F$ dans les imides simples par des groupements non perfluorés ou seulement partiellement fluorés diminue l'acidité et la solubilité d'une façon sensible.

Le but de la présente invention est de fournir de nouveaux composés ioniques dérivés de perfluorosulfonimides dans lesquels la délocalisation de la charge anionique est améliorée, ce qui entraîne une acidité et une dissociation nettement supérieures à celles des composés connus, tout en conservant une bonne stabilité.

C'est pourquoi la présente invention a pour objet des composés ioniques, et leurs applications.

Un composé selon l'invention est un composé ionique répondant à l'une des formules suivantes :

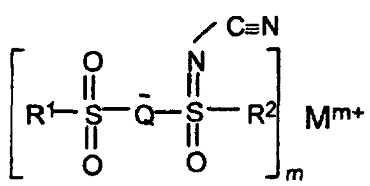

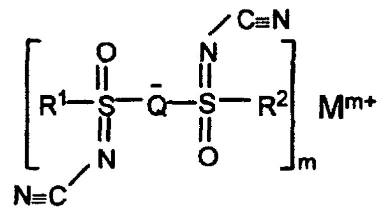

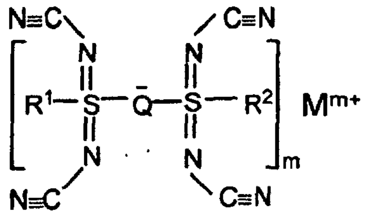

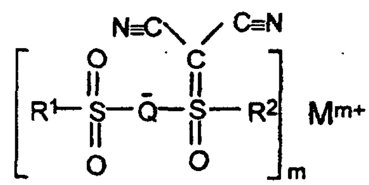

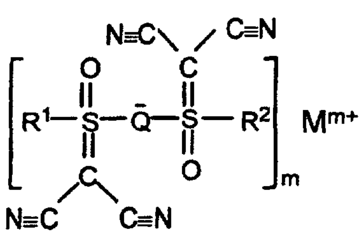

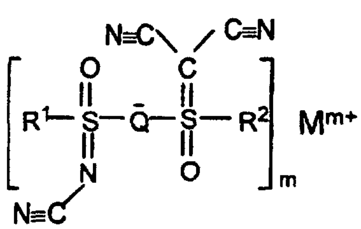

dans lesquelles

- $M^{m+}$ est le proton, ou un cation métallique ayant la valence m choisi parmi les ions des métaux alcalins, des métaux alcalino-terreux, des métaux de transition, des terres rares, ou un cation organique onium, ou un cation organo-métallique, $1 \leq m \leq 3$ ;
- Q représente N, $CR^5$, CCN ou $CSO_2R^5$ ;
- $R^1$, $R^2$, $R^3$, $R^4$, $R^5$ et $R^6$, désignés ci-après par $R^i$, représentent indépendamment les uns des autres Y, YO-, YS-, $Y_2N$- ou F ;
- Y représente un radical organique monovalent, ayant de préférence de 1 à 16 atomes de carbone, choisi parmi les radicaux alkyles, alkényles, oxa-alkyles, oxa-alkényles, aza-alkyles, aza-alkényles, aryles ou alkylaryles, perfluoroalkyles , ou parmi les radicaux obtenus à partir des radicaux précités par substitution, dans les chaînes et / ou la partie aromatique, par des hétéroatomes tels que les halogènes, l'oxygène, l'azote, le soufre, le phosphore ; étant entendu que **si** le soufre ou le phosphore sont présents, ils peuvent éventuellement être liés à des atomes d'oxygène ou d'azote substitués, ou bien Y est une unité de répétition d'une trame polymérique.

Lorsque $M^{m+}$ est un cation métallique, il peut être un métal alcalin (en particulier $Li^+$ et $K^+$), un métal alcalino-terreux (en particulier $Mg^{++}$, $Ca^{++}$ ou $Ba^{++}$), un métal de transition (en particulier $Cu^{++}$, $Zn^{++}$ ou $Fe^{++}$) ou une terre rare (en particulier $Re^{+++}$).

Lorsque $M^{m+}$ est un cation onium, il peut être choisi parmi les ions ammonium $[N(Y^j)_4]^+$, les ions amidinium $RC[N(Y^j)_2]^+$, les ions guanidinium $C[N(Y^j)_2]_3^+$, les ions pyridinium $C_5[N(Y^j)_6]^+$, les ions imidazolium $C_3N_2(Y^j)_4^+$, les ions imidazolinium $C_3N_2(Y^j)_7^+$, les ions triazolium $C_2N_3(Y^j)_4^+$, les ions carbonium $C_6(Y^j)_5C^+$, les ions $NO^+$ (nitrosyle) ou $NO_2^+$, les ions sulfonium $[S(Y^j)_3]^+$, les ions phosphonium $[P(Y^j)_4]^+$ et les ions iodonium $[I(Y^j)_2]^+$. Dans les différents ions onium précités, les substituants $Y^j$ d'un même anion peuvent être identiques ou différents. Ils représentent indépendamment les uns des autres H ou l'un des substituants indiqués ci-dessus pour Y.

Lorsque $M^{m+}$ est un cation organo-métallique, il peut être choisi parmi les métallocéniums. A titre d'exemple, on peut citer les cations dérivés du ferrocène, du titanocène, du zirconocène, d'un indénocénium ou d'un arène métallocénium. Il peut également être choisi parmi les cations métalliques coordonnés par des atomes tels que O, S, Se N, P ou As, portés par des molécules organiques, notamment sous forme de ligands carbonyle, phosphine ou porphyrine possédant éventuellement une chiralité. $M^{m+}$ peut également être un cation dérivé des groupements alkyles définis pour Y ci-dessus et limités à ceux ayant de 1 à 10 atomes de carbone, par exemple un dérivé trialkyl-silyle, tétraalkylgermanyle ou dialkyl-stannyle ; dans ce cas, M est relié au groupement $[R^1\text{-}X^1(Z^1)\text{-}Q^-\text{-}X^2(Z^2)\text{-}R^2]$ par une liaison covalente très labile et le composé se comporte comme un sel. Le cation $M^+$ peut également être l'unité de répétition d'un polymère conjugué sous forme oxydée cationique. Comme exemples particuliers, on peut citer les cations méthylzinc, phénylmercure, trialkylétain ou trialkylplomb, chloro[éthylènebis(indényl)] zirconium(IV) ou tétrakis(acétonitrile)palladium(II). Le cation organo-métallique peut faire partie d'une chaîne polymère.

Dans les composés de l'invention, la présence d'un ou plusieurs groupes $=N\text{-}C\equiv N$ ou $=C(C\equiv N)_2$ permet d'augmenter la dissociation et la résistance à l'oxydation de l'anion sans augmenter appréciablement sa masse et son volume molaire.

Le choix des substituants $R^i$ dans les composés de la présente invention permet d'obtenir des composés dans lesquels l'anion possède une chiralité intrinsèque sur un atome de soufre. De tels composés sont utiles pour induire une sélectivité éniantomérique lors de la préparation de composés organiques actifs ou pour induire une stéréosélectivité dans les réactions de polymérisation. Parmi ces composés, on peut citer ceux qui répondent à l'une ou l'autre des formules suivantes $[R^1SO_2N\text{-}S^*{=}O(R^2){=}NSO_2R^6]^-$ dans laquelle $R^1$ est différent de $R^6$, ou $[R^1SO_2N\text{-}S^*{=}O(R^2){=}N\text{-}S^*{=}O(R^5){=}NSO_2R^6]^-$. On préfère tout particulièrement les composés dans lesquels $R^1$ et $R^6$ représentent indépendamment l'un de l'autre un radical choisi parmi F, $CF_3$, $C_2F_5$, $C_4F_9$, $C_6F_{13}$ et $C_8F_{17}$, et $R^2$ et $R^5$ représentent indépendamment l'un de l'autre un alkyle, un aryle, un alkylaryle ou un dialkylamino ayant de préférence de 1 à 20 atomes de carbone.

Les composés ioniques de la présente invention peuvent être préparés par divers procédés.

De manière générale, un composé répondant à la formule

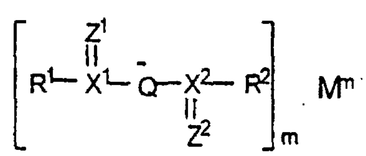

est préparé en faisant réagir un composé précurseur noté ci-après ($Z^1$ L) comprenant le groupe $Z^1$ et un groupe partant L sur un dérivé $A^2Z^2$ du groupe $Z^2$ selon l'un des schémas réactionnels suivants :

$$R^1\text{-}X^1(Z^1)\text{-}Q\text{-}X^2(L)R^2 + A^2Z^2 \Rightarrow [R^1\text{-}X^1(Z^1)\text{-}X^2(L)\,(Z_2)R^2]^- A^- + LA$$

ou

$$R^1\text{-}X^1(Z^1)\text{-}QA_2 + L\text{-}X^2(Z^2)R^2 \Rightarrow [R^1\text{-}X^1(Z^1)\text{-}X^2(L)\,(Z_2)R^2]^+ A^- + LA$$

A représente un métal alcalin, un proton, une base aminée ou phosphorée, un groupe trialkyl silyle, un groupe dialkyle stannyle, MgL1, ZnL1, CdL1, Cu, Mg, Zn, Cd, Hg, un groupement trialkyl-silyle, trialkyle-germanyle ou trialkyle-stannyle.

Les groupes partants L ou L1 sont avantageusement choisis parmi les halogènes, les pseudohalogènes incluant les sulfonates fluorés ou non, les radicaux imidazoyle, triazolyle et benzotriazoyle.

Les composés ($Z^1$ L) dans lesquels le groupe partant est un halogène peuvent être préparés par exemple par action d'un agent halogénant sur un sel $R^1\text{-}X^1(Z^1)\text{-}Q\text{-}X^2(O)(R^2)^-A^+$ ou sur l'acide correspondant. Le cation A est de préférence choisi parmi les cations de métal alcalin, les ions ammoniums inorganique $NH_4^+$ ou organiques $R^3NH^+$ (y compris le pyridinium) et l'ion $Ag^+$ qui présente une forte affinité pour Cl, Br, I.

Parmi les agents halogénants utiles, on peut citer $SF_4$, le trifluoro(diéthylamino)soufre IV (DAST), le chlorure de thionyle, le chlorure d'oxalyle, le fluorure d'oxalyle, le pentachlorure de phosphore, le mélange $P\Phi_3 + CCl_4$, le chlorure de (chlorométhylène)diméthyl ammonium $[CH(Cl)=N(CH_3)_2]^+Cl^-$ ou son homologue dérivé de la N-méthylpyrrolidinone, l'iodure de 1-méthyl-2-fluoro pyridinium. La préparation d'un composé ($Z^1$ L) dans lequel le groupe partant est un halogène est illustrée schématiquement par l'exemple suivant :

$$[CF_3SO_2NSO_2(C_4H_9)]Na + (COCl) \Rightarrow CO + CO_2 + [CF_3SO_2NSO(C_4H_9)Cl] + NaCl$$

Les composés précurseurs ($Z^1$ L) dans lesquels le groupe partant est' l'imidazolyle, le triazolyle ou le benzotriazolyle, peuvent être obtenus par action de leur sel alcalin, de leur dérivé du triméthyl-silyle ou de leur dérivé diméthylstannyle sur le composé précurseur halogéné correspondant, par exemple selon le schéma réactionnel suivant : $[CF_3SO_2NSO(C_4H_9)Cl] + ImSi(CH_3)_3 \Rightarrow ClSi(CH_3)_3 + [CF_3SO_2NSO(C_4H_9)Im]$, dans lequel Im représente

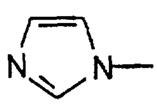
imidazolyle

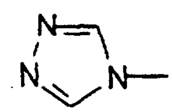
triazolyle

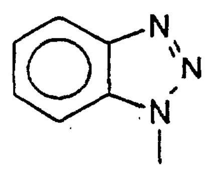
benzotriazolyle

Les composés précurseurs ($Z^1$ L) dans lesquels le groupe partant est un pseudo-halogène tel qu'un sulfonate peuvent être obtenus par action du chlorure d'acide ou de l'anhydride de l'acide sulfonique correspondant au sulfonate, sur un sel précité $R^1\text{-}X^1(Z^1)\text{-}Q\text{-}X^2(O)R^{2-}M^-$. La réaction d'un sel d'argent sur un chlorure de l'acide sulfonique $R^7SO^2Cl$ ($R^7$ étant de même nature que les $R^i$) selon le schéma suivant est particulièrement avantageuse : $R^1\text{-}X(Z^1)\text{-}Q\text{-}X(O)R^{2-}Ag^+ + R^7SO_2Cl \Rightarrow R^1\text{-}X(Z^1)\text{-}Q\text{-}X(SO_3R^7)R^2 + AgCl$

De manière générale, il est avantageux de préparer les composés précurseurs $R^1\text{-}X^1(Z^1)\text{-}Q\text{-}X^2(L)R^2$ à partir d'un composé comprenant un anion dans lequel le soufre ou le phosphore sont à l'état d'oxydation respectivement IV et III. Par oxydation de ces anions, les dérivés de soufre VI ou du phosphore V sont obtenus selon le schéma réactionnel.

$$R^1\text{-}X(Z^1)\text{-}Q\text{-}X^2(R^2)^-A^+ + 2L \Rightarrow R^1\text{-}X(Z^1)\text{-}Q\text{-}X^2(L)R^2 + LA$$

Les composés préférés pour L sont les halogènes, tels le fluor, le chlore, le brome.

Les différents groupes partants peuvent être échangés par des techniques bien connues de l'homme de

métier. Par exemple, le chlore peut être remplacé par le fluor par action d'un agent possédant des ions fluorure actifs et une affinité pour les ions chlore, tel que le fluorure d'argent AgF ou le fluorure de tétraméthyl ammonium, le fluorure de 1,1,1,3,3,3-hexakis(diméthylamino)diphosphazénium $\{[(CH_3)_2N]_3P\}_2N^+F^-$ ou le tétrakis(tris(diméthylamino)-phosphoranylidèneaminophosphonium $\{[(CH_3)_2N]_3P{=}N\}_4P^+F^-$, le composé d'addition du fluorure de tris-(diméthylamino) sulfonium avec le triméthylfluorosilane $[(CH_3)_2N]_3S^+[Si(CH_3)_3F_2]^-$.

Les dérivés de l'imidazole ou du triazole peuvent être obtenus par action de leur sel alcalin ou de leur dérivé du triméthyl-silyle ou du diméthyl-stannyle) sur le dérivé correpondant, selon le schéma réactionnel :

$$[CF_3SO_2NSO(C_4H_9)Cl] + ImSi(CH_3)_3 \Rightarrow$$

$$ClSi(CH_3)_3 + [CF_3SO_2NSO(C_4H_9)Im]$$

dans lequel Im représente l'imidazolyle, le triazolyle ou le benzotriazolyle.

Un composé symétrique, dans lequel $X^1(Z^1)$ est identique à $X^2(Z^2)$ peut être préparé par action d'un nitrure ionique ou d'un dérivé métallique d'hexaméthydisilazane ou d'ammoniac en présence d'une base sur un précurseur possédant un groupe partant L, selon le schéma réactionnel suivant, dans lequel R, X et Z ont la signification donnée ci-dessus respectivement pour $R^i$, $X^i$ et $Z^i$, A et L sont tels que définis ci-dessus : $2RX(Z)L + A_3N \Rightarrow [RX(Z)]_2N^-A^+ + 2LA$. Par exemple :

$$2CF_3SO(=NSO_2CF_3)F + Li_3N \Rightarrow [CF_3SO(=NSO_2CF_3)]_2N^-A^+ + 2LiF$$

L'agent nitrurant peut être avantageusement $Li_3N$, l'ammoniac, ses dérivés avec les silanes et leurs dérivés alcalins tels $N[SiCH_3)_3]_2Li$, $N[SiCH_3)_3]_2Na$ et $N[SiCH_3)_3]_2K$.

Un composé de formule $[R^1SO_2N\text{-}S^*{=}O(R^2){=}NSO_2R^3]^-\ M^+$ peut être obtenu en faisant réagir un sel $[R^1SO_2NSO_2R^2]^-\ M'^+$ avec un agent halogénant, pour obtenir le précurseur $R^1SO_2NSOR^2(X)$ (X étant un halogène). Ledit précurseur est ensuite condensé sur une sulfonamide $R^3SO_2NH_2$ en présence d'une base ou sur ses dérivés métalliques comme $R^3SO_2NLi_1$ ou $R^3SO_2NNa_2$. Le cation souhaité pour le composé final est obtenu par des procédés classiques d'échange d'ions.

De la même manière, les carbures ioniques permettent de préparer les composés $[RX(Z)]_3C^-A^+$, selon le schéma réactionnel $2RX(Z)L + A_4C \Rightarrow [RX(Z)]_3C^-A^+ + 3LA$.

Compte tenu du grand choix possible pour les substituants qui peuvent être présents sur le groupement anionique, les composés de l'invention permettent d'induire des propriétés de conduction ionique dans la plupart des milieux organiques, liquides ou polymères possédant une polarité, même faible. Les applications sont importantes dans le domaine de l'électrochimie, en particulier du stockage de l'énergie dans des générateurs primaires ou secondaires, dans les supercapacités, dans les piles à combustibles et dans les diodes électroluminescentes. La compatibilité des composés ioniques de l'invention avec les polymères ou les liquides organiques permet d'induire des propriétés antistatiques marquées, même lorsque la teneur en composé ionique est extrêmement faible. C'est pourquoi un autre objet de la présente invention est constitué par un matériau à conduction ionique constitué par un composé ionique de la présente invention en solution dans un solvant.

Le composé ionique utilisé pour l'élaboration d'un matériau à conduction ionique est de préférence choisi parmi les composés dont le cation est l'ammonium, ou un cation dérivé d'un métal, en particulier le lithium ou le potassium, le zinc, le calcium, les métaux des terres rares, ou un cation organique, tel qu'un ammonium substitué, un imidazolium, un triazolium, un pyridinium, un 4-diméthylamino-pyridinium, lesdits cations portant éventuellement un substituant sur les atomes de carbone du cycle. Le matériau à conduction ionique ainsi obtenu présente une conductivité et une solubilité élevées dans les solvants, du fait des interactions faibles entre la charge positive et la charge négative. Son domaine de stabilité électrochimique est étendu, et il est stable dans des milieux aussi bien réducteurs qu'oxydants. De plus, les composés qui ont un cation organique et un point de fusion inférieur à 150°C, en particulier les composés ayant un cation imidazolium, triazolium, pyridinium, ou 4-diméthylamino-pyridinium présentent une conductivité élevée intrinsèque, même en l'absence de solvant, lorsqu'ils sont en phase fondue.

Le solvant d'un matériau à conduction ionique de l'invention peut être un solvant liquide aprotique, un polymère solvatant, un polymère polaire ou un de leurs mélanges. Le solvant liquide aprotique est choisi par exemple parmi les éthers linéaires et les éthers cycliques, les esters, les nitriles, les dérivés nitrés, les amides, les sulfones, les sulfolanes, les alkylsulfamides et les hydrocarbures partiellement hydrogénés. Les solvants particulièrement préférés sont le diéthyléther, le diméthoxyéthane, le glyme, le tétrahydrofurane, le dioxane, le diméthyltétrahydrofurane, le formiate de méthyle ou d'éthyle, le carbonate de propylène ou d'éthylène, les carbonates d'alkyle (notamment le carbonate de diméthyle, le carbonate de diéthyle et le carbonate de méthylpropyle), les butyrolactones, l'acétonitrile, le benzonitrile,

le nitrométhane, le nitrobenzène, la diméthylformamide, la diéthylformamide, la N-méthylpyrrolidone, la diméthylsulfone, la tétraméthylène sulfone, la tétraméthylène sulfone et les tétraalkylsulfonamides ayant de 5 à 10 atomes de carbone.

Le solvant du matériau à conduction ionique peut être un polymère polaire choisi parmi les polymères solvatants, réticulés ou non, portant ou non des groupes ioniques greffés. Un polymère solvatant est un polymère qui comporte des unités solvatantes contenant au moins un hétéroatome choisi parmi le soufre, l'oxygène, l'azote et le fluor. A titre d'exemple de polymères solvatants, on peut citer les polyéthers de structure linéaire, peigne ou à blocs, formant ou non un réseau, à base de poly(oxyde d'éthylène), ou les copolymères contenant le motif oxyde d'éthylène ou oxyde de propylène ou allylglycidyléther, les polyphosphazènes, les réseaux réticulés à base de polyéthylène glycol réticulé par des isocyanates ou les réseaux obtenus par polycondensation et portant des groupements qui permettent l'incorporation de groupements réticulables. On peut également citer les copolymères à blocs dans lesquels certains blocs portent des fonctions qui ont des propriétés rédox. Bien entendu, la liste ci-dessus n'est pas limitative, et tous les polymères présentant des propriétés solvatantes peuvent être utilisés.

Un matériau à conduction ionique de la présente invention peut comprendre simultanément un solvant liquide aprotique choisi parmi les solvants liquides aprotiques cités ci-dessus et un solvant polymère polaire comprenant des unités contenant au moins un hétéroatome choisi parmi le soufre, l'azote, l'oxygène et le fluor. Il peut comprendre de 2 à 98% de solvant liquide. A titre d'exemple d'un tel polymère polaire, on peut citer les polymères qui contiennent principalement des unités dérivées de l'acrylonitrile, du fluorure de vinylidène, de la N-vinyipyrrolidone ou du méthacrylate de méthyle. Ces polymères peuvent porter des groupements ioniques. La proportion de liquide aprotique dans le solvant peut varier de 2% (correspondant à un solvant plastifié) à 98% (correspondant à une solvant gélifié). Un matériau à conduction ionique de la présente invention peut contenir en outre un sel utilisé classiquement dans l'art antérieur pour l'élaboration d'un matériau à conduction ionique. Parmi les sels utilisables en mélange avec un composé ionique selon l'invention, on préfère tout particulièrement un sel choisi parmi les perfluoroalcanesulfonates, les bis (perfluoroalkylsulfonyl) imidures, les bis(perfluoroalkylsulfonyl) méthanes et les tris(perfluoroalkylsulfonyl)méthanes.

Bien entendu, un matériau à conduction ionique de l'invention peut contenir en outre les additifs utilisés de manière classique dans ce type de matériau, et notamment des charges minérales ou organiques sous forme de poudre ou de fibres.

Un matériau à conduction ionique de l'invention peut être utilisé comme électrolyte dans un générateur électrochimique. La présente invention a ainsi pour autre objet un générateur électrochimique comprenant une électrode négative et une électrode positive séparées par un électrolyte, caractérisé en ce que l'électrolyte est un matériau à conduction ionique tel que défini ci-dessus. De préférence, le cation du composé ionique de l'électrolyte est $Li^+$ ou $K^+$. Selon un mode de réalisation particulier, un tel générateur comprend une électrode négative constituée par du lithium métallique, ou par l'un de ses alliages, éventuellement sous forme de dispersion manométrique dans de l'oxyde de lithium, ou par un nitrure double de lithium et d'un métal de transition, ou par un oxyde à bas potentiel ayant pour formule générale $Li_{1+y}Ti_{2-x/4}O_4$ ($0 \leq x, y \leq 1$), ou par le carbone et les produits carbonés issus de la pyrolyse de matières organiques. Lorsque l'électrode négative fonctionne par échange d'ions lithium, il est particulièrement intéressant d'utiliser pour l'électrolyte, un composé de l'invention dans lequel le cation est un ion $Li^+$. Selon un autre mode de réalisation, le générateur comprend une électrode positive choisie parmi les oxydes de vanadium $VO_x$ ($2 \leq x \leq 2{,}5$), $LiV_3O_8$, $Li_yNi_{1-x}Co_xO_2$, ($0 \leq x, y \leq 1$), les spinelles de manganèse $Li_yMn_{1-x}M_xO_2$ (M = Cr, Al, V, Ni, $0 \leq x \leq 0{,}5$ ; $0 \leq y \leq 2$), les polydisulfures organiques, FeS, $FeS_2$, le sulfate de fer $Fe_2(SO_4)_3$, les phosphates et phosphosilicates de fer et de lithium de structure olivine, ou leurs produits de substitution du fer par le manganèse, utilisés seuls ou en mélanges. Le collecteur de l'électrode positive est de préférence en aluminium.

Un composé ionique de la présente invention peut également être utilisé pour induire une conductivité ionique dans des milieux de faible polarité, tels que les hydrocarbures aliphatiques et aromatiques et les milieux qui en contiennent une fraction importante, les polymères à caractère peu polaire et / ou hydrophobes, et le dioxyde de carbone supercritique.

Un matériau à conduction ionique de la présente invention peut également être utilisé dans une supercapacité. Un autre objet de la présente invention est par conséquent une supercapacité utilisant au moins une électrode de carbone à haute surface spécifique, ou une électrode contenant un polymère rédox, dans laquelle l'électrolyte est un matériau à conduction ionique tel que défini ci-dessus.

Les composés ioniques de la présente invention peuvent être utilisés pour dopage de polymères en vue de leur conférer une conduction électronique améliorée. Les polymères concernés sont essentiellement les polyacétylènes, les polyphénylènes, les polypyrroles, les polythiophènes, les polyanilines, les polyquinolines substitués ou non, ainsi que les polymères dans lesquels les motifs aromatiques sont séparés par le motif vinylène -CH=CH-. Le procédé de dopage consiste à oxyder partiellement le polymère pour créer des carbocations dont la charge est compensée par les anions des composés de l'invention. Ce dopage peut être effectué de façon chimique ou électrochimique, éventuellement simultanément à la formation du polymère. Pour cette application particulière, on choisit de préférence les composés de l'invention portant un charge très délocalisée, en particulier les composés dans lesquels Z est =C

$(C{\equiv}N)_2$ $=NSO_2R$ ou $=C(SO_2R)_2$ qui confèrent des propriétés de stabilité thermique et mécanique. Les polymères ainsi dopés sont un autre objet de la présente invention.

En outre, un matériau à conduction ionique de la présente invention peut être utilisé comme électrolyte dans un dispositif électrochrome. Un dispositif électrochrome dans lequel l'électrolyte est un matériau à conduction ionique selon l'invention est un autre objet de la présente invention. Un tel dispositif comprend en outre des électrodes dont la matière active est choisie parmi $WO_3$, $MoO_3$, les oxyhydroxydes d'iridium $IrO_xH_y$, ($2 \leq x \leq 3$ ; $0 \leq y \leq 3$), le bleu de prusse, les viologènes et leurs polymères, et les polyimides aromatiques.

Les composés de la présente invention peuvent être utilisés pour la catalyse de divers types de réactions chimiques, et notamment pour des réactions de polymérisation, des réactions de condensation, des réactions d'addition ou d'élimination, des réactions d'oxydation ou de réduction, des solvolyses, des réactions de Friedel et Craft et des réactions de Diels Alder. Pour ces applications en catalyse, les composés seront choisis essentiellement en fonction du cation associé à la partie anionique.

Four la catalyse de réactions de Diels Alder ou de réactions de Friedel et Crafts, les cations de métal alcalin, de métal alcalino-terreux, de métal de transition ou de terre rare conviennent. Les composés ayant un cation $H^+$, $Li^+$, $Mg^{++}$, $Ca^{++}$, $Cu^{++}$, $Zn^{++}$, $Al^{+++}$, $Fe^{+++}$ ou $Fe^{+++}$ sont préférés.

Les composés de l'invention dans lesquels le cation est un onium du type diazonium, sulfonium, iodonium, métallocénium, peuvent être utilisés comme amorceurs de polymérisation cationique, notamment pour polymériser ou réticuler les éthers vinyliques, les époxydes, les acétals et éthers cycliques, les vinylamides, les oxazolines, l'isobutylène, le styrène ou les siloxanes. Sous l'action d'un rayonnement actinique, de tels composés génèrent la forme acide correspondante capable d'amorcer une réaction de polymérisation cationique. Un composé de l'invention peut être utilisé en tant que photoamorceur éventuellement en présence d'un sensibilisateur, ou d'un amorceur radicalaire amorçable thermiquement ou par un rayonnement actinique. Les composés de l'invention sous forme de sel d'amine peuvent servir d'amorceur des polymérisations cationiques par chauffage libérant la forme protonique correspondante. De même, si le cation est un sel d'un composé azoïque cationique (par exemple tel que représenté ci-dessous) il peut servir par chauffage d'amorceur des polymérisations radicalaires.

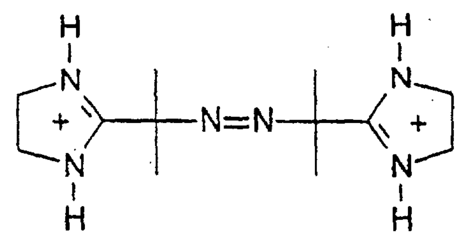

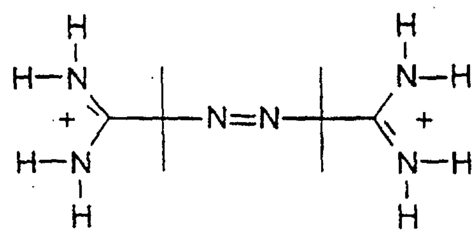

La présente invention permet d'obtenir des composés dans lesquels l'anion présente une chiralité intrinsèque, ce qui permet d'induire des asymétries énantiomériques lors de l'utilisation desdits composés en tant que catalyseurs, de préparer des polymères stéréoréguliers et de conférer un pouvoir rotatoire aux matériaux les contenant.

La présente invention est expliquée plus en détails par les exemples suivants, qui décrivent la préparation et diverses utilisations de composés de l'invention. L'invention n'est toutefois pas limitée à ces exemples.

**Exemple 1**

Le composé ionique $[CF_3SO_2NSO_2(3,5\text{-}C_6H_3(CF_3)_2]^-K^+$ a été préparé par une méthode similaire à celle de l'exemple 2 à partir de chlorure de 3,5-bis(trifluorométhyl)benzène sulfonyle et de trifluométhane sulfonamide. 18,6 g de sel ont été traités par 7 g de DAST $(C_2H_5)_2NSF_3$. Le composé fluoré obtenu :

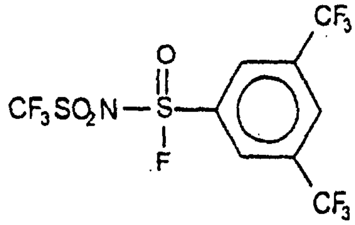

a été purifié par distillation sous vide. 4,27 g de ce composé ont été ajoutés à 30 ml de THF anhydre contenant 670 mg de malononitrile et 18 mg d'hydrure de lithium. A la fin de la réaction, observée par la fin du dégagement d'hydrogène, le mélange réactionnel a été filtré et le THF évaporé. Le résidu solide a été repris par l'eau et filtré. On a ajouté 4,4 g de sulfate de brucine dans 50 ml d'eau et le mélange réactionnel a été agité pendant 24 heures. Après séparation et séchage, 8 g du précipité formé ont été traités par une solution de 1,6 g d'une solution de tétraphénylborate de sodium dans 20 ml d'une solution équivolumique eau-éthanol. La solution après filtration a été séchée pour donner le sel de sodium de l'anion intrinsèquement chiral par son atome de soufre, résolu en isomère actif par la brucine :

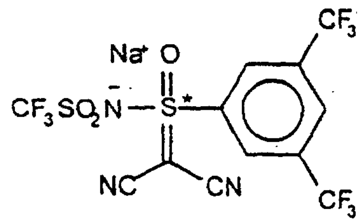

Les sels de lithium, de magnésium ou de terres rares et d'yttrium de cet anion induisent des excès énantiomériques de 50 à 92% lors de la catalyse des réactions de Diels Alder et des condensations aldoliques. Un catalyseur de polymérisation cationique a été préparé par action du chlorure d'acétyle en quantité stoechiométrique sur le sel d'argent, lui-même obtenu par échange du sel de sodium par le toluène-sulfonate d'argent dans un mélange isopropanoltoluène. Ce catalyseur induit une polymérisation de l'oxyde de propylène en un polymère optiquement actif. D'une façon similaire, l'éther méthyl-vinylique est polymérisé en une macromolécule isotactique cristalline insoluble dans l'eau, contrairement aux polymères obtenus avec les amorceurs cationiques non chiraux. Les composés :

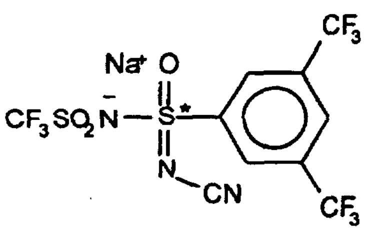

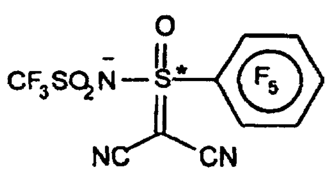

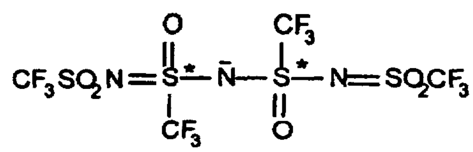

présentent aussi une chiralité intrinsèque sur le centre anionique, permettant la catalyse de réactions favorisant un éniantomère, les polymérisations donnant des polymères optiquement actifs ou présentant une tacticité.

## Revendications

**1.** Composé ionique répondant à l'une des formules suivantes :

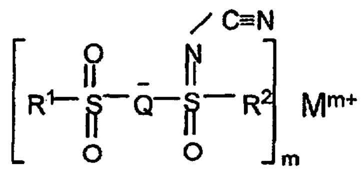

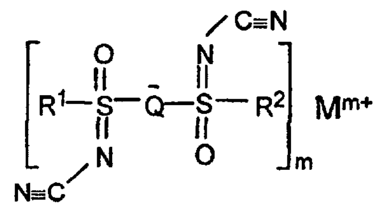

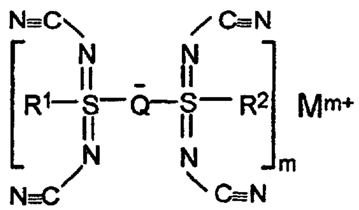

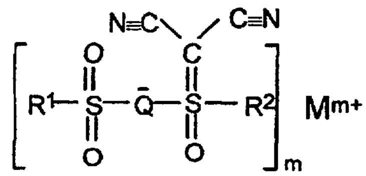

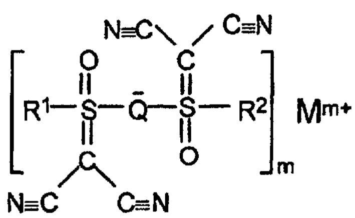

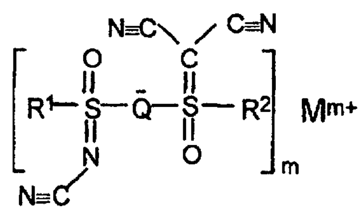

dans lesquelles :

- $M^{m+}$ est le proton, ou un cation métallique ayant la valence m choisi parmi les ions des métaux alcalins, des métaux alcalino-terreux, des métaux de transition, des terres rares, ou un cation organique onium, ou un cation organo-métallique, $1 \leq m \leq 3$ ;
- Q représente N, $CR^5$, CCN ou $CSO_2R^5$ ;
- $R^1$, $R^2$, et $R^5$, désignés ci-après par $R^i$, représentent indépendamment les uns des autres Y, YO-, YS-, $Y_2N$- ou F ;
- Y représente un radical organique monovalent, choisi parmi les radicaux alkyles, alkényles, oxa-alkyles, oxa-alkényles, aza-alkyles, aza-alkényles, aryles ou alkylaryles, perfluoroalkyles , ou parmi les radicaux obtenus à partir des radicaux précités par substitution, dans les chaînes et / ou la partie aromatique, par des hétéroatomes étant entendu que **si** le soufre ou le phosphore sont présents, ils peuvent éventuellement être liés à des atomes d'oxygène ou d'azote substitués, ou bien Y est une unité de répétition d'une trame polymérique.

**2.** Composé ionique selon la revendication 1, **caractérisé en ce que** le cation $M^{m+}$ est un cation métallique choisi parmi $K^+$, $Li^+$, $Mg^{++}$, $Ca^{++}$ ou $Ba^{++}$, $Cu^{++}$, $Zn^{++}$, $Fe^{++}$ ou $Re^{+++}$.

**3.** Composé ionique selon la revendication 1, **caractérisé en ce que** le cation $M^{m+}$ est un cation onium choisi dans le groupe constitué par les ions ammonium, les ions guanidinium, les ions amidinium, les ions pyridinium, les ions imidazolium, les ions imidazolinium, les ions triazolium, les ions sulfonium, les ions iodonium, les ions phosphonium, le nitrosyle et $NO_2^+$.

**4.** Composé ionique selon la revendication 1, **caractérisé en ce que** le cation $M^{m+}$ est cation organo-métallique choisi parmi les ions métallocénium, les cations métalliques coordonnés par des atomes O, S, Se N, P ou As et portés par des molécules organiques, les groupes trialkyl-silyle, tétraalkyl-germanyle et dialkyl-stannyle dans lesquels les radicaux alkyles ont de 1 à 10 atomes de carbone.

**5.** Composé ionique selon la revendication 4, **caractérisé en ce que** le cation organo-métallique fait partie d'une

chaîne polymère.

6. Composé ionique selon la revendication 1, **caractérisé en ce que** les radicaux $R^1$ et $R^2$ sont choisis indépendamment l'un de l'autre parmi les radicaux perfluoroalkyles, les radicaux alkyles, les radicaux alkényle, les radicaux dialkylamino et les radicaux styrényles.

7. Composé ionique selon la revendication 6, **caractérisé en ce que** les radicaux perfluoroalkyles ont de 1 à 8 atomes de carbone, les radicaux alkyles ont de 1 à 8 atome de carbone, les radicaux alkényle ont de 2 à 18 atomes de carbone, les radicaux alkyles des groupements dialkylamino ont de 1 à 18 atomes de carbone.

8. Matériau à conduction ionique constitué par une solution d'un composé ionique selon la revendication 1 dans un solvant.

9. Matériau selon la revendication 8, **caractérisé en ce que** le cation du composé ionique est choisi parmi l'ammonium, le lithium, le potassium, le zinc, le calcium, un ammonium substitué, un imidazolium, un triazolium, un pyridinium, un 4-diméthylamino-pyridinium, lesdits cations portant éventuellement un substituant sur les atomes de carbone du cycle.

10. Matériau selon la revendication 8, **caractérisé en ce que** le solvant est un solvant liquide aprotique.

11. Matériau à conduction ionique selon la revendication 8, **caractérisé en ce que** le solvant est un polymère solvatant, réticulé ou non, portant ou non des groupes ioniques greffés.

12. Matériau à conduction ionique selon la revendication 8, **caractérisé en ce que** le solvant est un mélange d'un solvant liquide aprotique et d'un polymère polaire.

13. Electrolyte constitué par un composé ionique en solution dans un solvant, **caractérisé en ce que** le composé ionique est un composé selon la revendication 1.

14. Batterie primaire ou secondaire, comprenant une électrode négative, une électrode positive et un électrolyte, **caractérisée en ce que** l'électrolyte contient un composé ionique selon la revendication 1.

15. Batterie primaire ou secondaire, comprenant une électrode négative, une électrode positive et un électrolyte, **caractérisée en ce que** l'électrolyte est un matériau à conduction ionique selon l'une des revendications 8 à 12.

16. Système de modulation de la lumière comprenant un électrolyte et des électrodes, **caractérisé en ce que** l'électrolyte contient un composé ionique selon la revendication 1.

17. Supercapacité constituée par un élecrolyte et des électrodes, **caractérisée en ce que** l'électrolyte contient un composé ionique selon la revendication 1.

18. Procédé pour le dopage d'un polymère consistant à oxyder partiellement ledit polymère pour créer des carbocations dont la charge est compensée par les anions d'un composé ionique, **caractérisé en ce que** le composé ionique est un composé selon la revendication 1.

19. Procédé de polymérisation ou de réticulation de monomères ou de prépolymères capables de réagir par voie cationique, **caractérisé en ce que** l'on utilise un composé selon la revendication 1 comme photoinitiateur source d'acide catalysant la réaction.

20. Utilisation d'un composé ionique selon la revendication 1 en tant que catalyseur des réactions de polymérisation, des réactions de condensation, des réactions d'addition ou d'élimination, des réactions d'oxydation ou de réduction, des solvolyses, des réactions de Friedel et Craft et des réactions de Diels Alder.

**Patentansprüche**

1. Ionische Verbindung, entsprechend einer der folgenden Formeln:

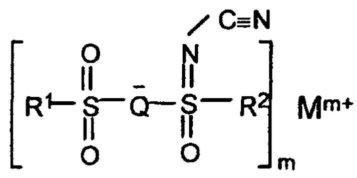

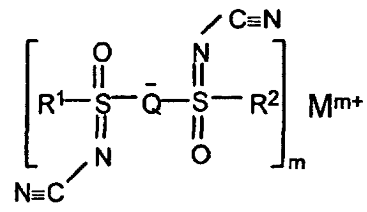

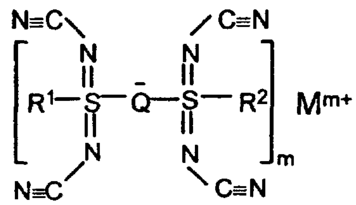

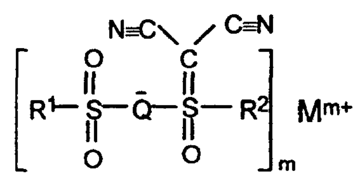

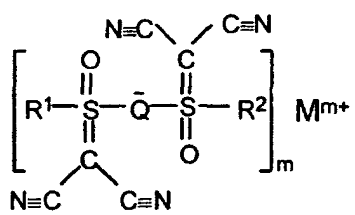

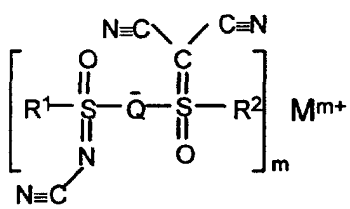

worin:

- $M^{m+}$ ein Proton oder ein Metallkation mit der Wertigkeit m, ausgewählt aus Alkalimetall-, Erdalkalimetall-, Übergangsmetall- und Seltenerdelementionen, oder ein organisches Onium-Kation oder ein organometallisches Kation ist, wobei gilt: $1 \leq m \leq 3$;
- Q für N, $CR^5$, CCN oder $CSO_2R^5$ steht;
- $R^1$, $R^2$ und $R^5$, in der Folge mit $R^i$ bezeichnet, jeweils unabhängig voneinander für Y, YO-, YS-, $Y_2N$- oder F stehen;
- Y für einen einwertigen organisch Rest steht, der aus Alkyl-, Alkenyl-, Oxaalkyl-, Oxaalkenyl-, Azaalkyl-, Azaalkenyl-, Aryl- oder Alkylaryl-, Perfluoralkylresten oder aus Resten ausgewählt ist, die durch Substitution der oben genannten Reste an den Heteroatomen in den Ketten und/oder dem aromatischen Abschnitt erhalten werden, wobei es sich versteht, dass, wenn Schwefel oder Phosphor vorhanden sind, diese gegebenenfalls mit substituierten Sauerstoff- oder Stickstoffatomen verbunden sein können, oder aber Y eine Grundeinheit eines Polymergerüsts ist.

**2.** Ionische Verbindung nach Anspruch 1, **dadurch gekennzeichnet, dass** das Kation $M^{m+}$ ein Metallkation, ausgewählt aus $K^+$, $Li^+$, $Mg^{++}$, $Ca^{++}$ oder $Ba^{++}$, $Cu^{++}$, $Zn^{++}$, $Fe^{++}$ oder $Re^{+++}$, ist.

**3.** Ionische Verbindung nach Anspruch 1, **dadurch gekennzeichnet, dass** das Kation $M^{m+}$ ein Oniumkation ist, das aus der aus Ammoniumionen, Guanidiniumionen, Amidiniumionen, Pyridiniumionen, Imidazoliumionen, Imidazoliniumionen, Triazoliumionen, Sulfoniumionen, Iodoniumionen, Phosphoniumionen, Nitrosyl und $NO_2^+$ bestehenden Gruppe ausgewählt ist.

**4.** Ionische Verbindung nach Anspruch 1, **dadurch gekennzeichnet, dass** das Kation $M^{m+}$ ein organometallisches Kation ist, das aus Metalloceniumionen, mit Atomen wie O, S, Se, N, P oder As koordinierten und von organisches Molekülen getragenen Metallkationen, Trialkylsilyl-, Tetraalkylgermanyl- und Dialkylstannylgruppen, worin die Alkylreste 1 bis 10 Kohlenstoffatome aufweisen, ausgewählt ist.

**5.** Ionische Verbindung nach Anspruch 4, **dadurch gekennzeichnet, dass** das organometallische Kation Teil einer

Polymerkette ist.

6. Ionische Verbindung nach Anspruch 1, **dadurch gekennzeichnet, dass** die Reste $R^1$ und $R^2$ unabhängig voneinander aus Perfluoralkylresten, Alkylresten, Alkenylresten, Dialkylaminoresten und Styrenylresten ausgewählt sind.

7. Ionische Verbindung nach Anspruch 6, **dadurch gekennzeichnet, dass** die Perfluoralkylreste 1 bis 8 Kohlenstoffatome aufweisen, die Alkylreste 1 bis 8 Kohlenstoffatome aufweisen, die Alkenylreste 2 bis 18 Kohlenstoffatome aufweisen und die Alkylreste der Dialkylaminogruppen 1 bis 18 Kohlenstoffatome aufweisen.

8. Ionenleitendes Material, das aus einer Lösung einer ionischen Verbindung nach Anspruch 1 in einem Lösungsmittel besteht.

9. Material nach Anspruch 8, **dadurch gekennzeichnet, dass** das Kation der ionischen Verbindung aus Ammonium, Lithium, Kalium, Zink, Kalzium, substituiertem Ammonium, Imidazolium, Triazolium, Pyridinium und 4-Dimethylaminopyridinium ausgewählt ist, wobei die Kationen gegebenenfalls einen Substituenten auf den Ring-Kohlenstoffatomen aufweisen.

10. Material nach Anspruch 8, **dadurch gekennzeichnet, dass** das Lösungsmittel ein flüssiges aprotisches Lösungsmittel ist.

11. Ionenleitendes Material nach Anspruch 8, **dadurch gekennzeichnet, dass** das Lösungsmittel ein vernetztes oder nicht vernetztes solvatisierendes Polymer ist, das gegebenenfalls aufgepfropfte ionische Gruppen aufweist.

12. Ionenleitendes Material nach Anspruch 8, **dadurch gekennzeichnet, dass** das Lösungsmittel ein Gemisch aus einem aprotischen flüssigen Lösungsmittel und einem polaren Polymer ist.

13. Elektrolyt, der aus einer ionischen Verbindung in Lösung in einem Lösungsmittel besteht, **dadurch gekennzeichnet, dass** die ionische Verbindung eine Verbindung nach Anspruch 1 ist.

14. Primär- oder Sekundärbatterie, die eine negative Elektrode, eine positive Elektrode und einen Elektrolyten umfasst, **dadurch gekennzeichnet, dass** der Elektrolyt eine ionische Verbindung nach Anspruch 1 enthält.

15. Primär- oder Sekundärbatterie, die eine negative Elektrode, eine positive Elektrode und einen Elektrolyten umfasst, **dadurch gekennzeichnet, dass** der Elektrolyt ein ionenleitendes Material nach einem der Ansprüche 8 bis 12 ist.

16. Lichtmodulationssystem, das einen Elektrolyten und Elektroden umfasst, **dadurch gekennzeichnet, dass** der Elektrolyt eine ionische Verbindung nach Anspruch 1 enthält.

17. Superkapazität, die aus einem Elektrolyten und Elektroden besteht, **dadurch gekennzeichnet, dass** der Elektrolyt eine ionische Verbindung nach Anspruch 1 enthält.

18. Verfahren zum Dotieren eines Polymers, das darin besteht, das Polymer teilweise zu oxidieren, um Carbokationen zu erzeugen, deren Ladung durch die Anionen einer ionischen Verbindung kompensiert wird, **dadurch gekennzeichnet, dass** die ionische Verbindung eine Verbindung nach Anspruch 1 ist.

19. Verfahren zur Polymersation oder Vernetzung von Monomeren oder Präpolymeren, die auf kationischem Weg reagieren können, **dadurch gekennzeichnet, dass** eine Verbindung nach Anspruch 1 als Photoinitator-Säurequelle dient, die die Reaktion katalysiert.

20. Verwendung einer ionischen Verbindung nach Anspruch 1 als Katalysator für Polymerisationsreaktionen, Kondensationsreaktionen, Additions- oder Eliminierungsreaktionen, Oxidations- oder Reduktionsreaktionen, Solvolysen, Friedel-Crafts-Reaktionen sowie Diels-Alder-Reaktionen.

**Claims**

1. Ionic compound corresponding to one of the following formulae

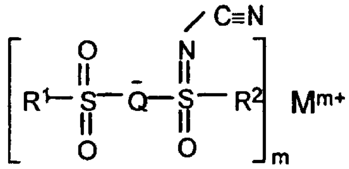

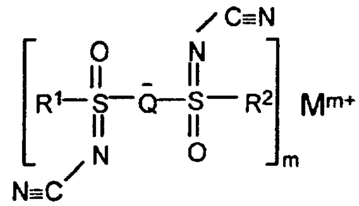

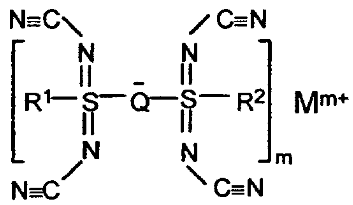

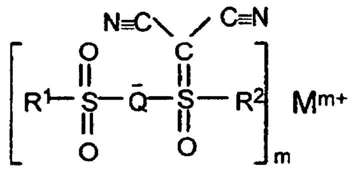

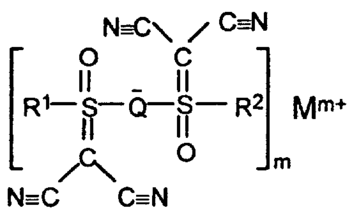

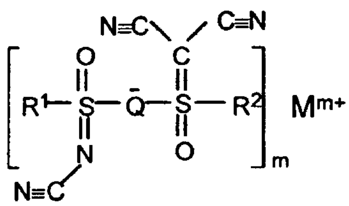

in which :

- $M^{m+}$ is a proton or a metal cation having the valency m, chosen from ions of alkali metals, of alkaline-earth metals, of transition metals or of rare-earth metals, or an organic onium cation or an organometallic cation, $1 \leq m \leq 3$ ;
- Q represents N, $CR^5$, CCN or $CSO_2R^5$ ;
- $R^1$, $R^2$, and $R^5$, denoted below by $R^i$, represent, independently of each other, Y, YO-, YS-, $Y_2N$- or F ;
- Y represents a monovalent organic radical chosen from alkyl, alkenyl, oxaalkyl, oxaalkenyl, azaalkyl, azaalkenyl, aryl or alkylaryl, perfluoroalkyl radicals, or from the radicals obtained from the abovementioned radicals by substitution, in the chains and/or the aromatic part, with hetero atoms, with the proviso that if sulfur or phosphorus are present, they may optionally be linked to oxygene atoms or substituted nitrogen atoms, or alternatively Y is a repeating unit of a polymeric backbone.

2. Ionic compound according to Claim 1, **characterized in that** the cation $M^{m+}$ is a metal cation chosen from $K^+$, $Li^+$, $Mg^{++}$, $Ca^{++}$ or $Ba^{++}$, $Cu^{++}$, $Zn^{++}$, $Fe^{++}$ or $Re^{+++}$.

3. Ionic compound according to Claim 1, **characterized in that** the cation $M^{m+}$ is an onium cation selected from the group consisting of ammonium ions, guanidinium ions, amidinium ions, pyridinium ions, imidazolium ions, imidazolinium ions, triazolium ions, sulfonium ions, iodonium ions, phosphonium ions, nitrosyl and $NO_2^+$.

4. Ionic compound according to Claim 1, **characterized in that** the cation $M^{m+}$ is an organometallic cation selected from metallocenium ions, metal cations coordinated by O, S, Se, N, P or As atoms and borne by organic molecules, trialkylsilyl, tetraalkylgermanyl or dialkylstannyl groups in which the alkyl radicals contain from 1 to 10 carbon atoms.

5. Ionic compound according to Claim 4, **characterized in that** the organometallic cation forms part of a polymer chain.

6. Ionic compound according to Claim 1, **characterized in that** the radicals $R^1$ and $R^2$ are chosen, independently of

each other, from perfluoroalkyl radicals, alkyl radicals, alkenyl radicals, dialkylamino radicals and styrenyl radicals.

**7.** Ionic compound according to claim 6, **characterized in that** the perfluoroalkyl radicals have from 1 to 8 carbon atoms, the alkyl radicals have from 1 to 8 carbon atoms, the alkenyl radicals have from 2 to 8 carbon atoms, the alkyl radicals of the dialkyamino groups have from 1 to 18 carbon atoms.

**8.** Ion conducting material consisting of a solution of an ionic compound according to Claim 1, in a solvent.

**9.** Material according to Claim 8, **characterized in that** the cation of the ionic compound is selected from ammonium, lithium, potassium, zinc, calcium, a substituted ammonium, an imidazolium, a triazolium, a pyridinium, 4-dimethylaminopyridinium, said cations optionally bearing a substituent on the carbon atoms of the ring.

**10.** Material according to Claim 8, **characterized in that** the solvent is an aprotic liquid solvent.

**11.** Ion conducting material according to claim 8, **characterized in that** the solvent is a crosslinked or non-crosslinked solvating polymer optionally bearing grafted ionic groups.

**12.** Ion conducting material according to claim 8, **characterized in that** the solvent is a mixture of an aprotic liquid solvent and a polar polymer.

**13.** An electrolyte consisting of ionic compound is solution in a solvent, **characterized in that** the ionic compound is a compound according to Claim 1.

**14.** A primary or secondary battery, comprising a negative electrode, a positive electrode and an electrolyte, **characterized in that** the electrolyte contains an ionic compound according to claim 1.

**15.** A primary or secondary battery, comprising a negative electrode, a positive electrode and an electrolyte, **characterized in that** the electrolyte is an ion conducting material according to any of Claim 8 to 12.

**16.** A system for modulating light, comprising an electrolyte and electrodes, **characterized in that** the electrolyte contains an ionic compound according to Claim 1.

**17.** A supercapacitor consisting of an electrolyte and electrodes, **characterized in that** the electrolyte contains an ionic compound according to claim 1.

**18.** A method for doping a polymer, consisting in partially oxidizing said polymer in order to create carbocations the charge of which is compensated by the anions of an ionic compound, **characterized in that** the ionic compound is a compound according to Claim 1.

**19.** Process for polymerizing or crosslinking monomers or prepolymers capable of reacting cationically, **characterized in that** a compound according to Claim 1 is used as a photoinitiating source of acid for catalyzing the reaction.

**20.** Use of an ionic compound according to Claim 1, as a catalyst for polymerization reactions, for condensation reactions, for addition or elimination reactions, for oxidation or reduction reactions, for solvolyses, for Friedel-Crafts reactions and for Diels-Alder reactions.